# EUROPEAN PATENT SPECIFICATION

(11) **EP 1 014 926 B1**
(45) Date of publication and mention of the grant of the patent: **04.02.2004**
(21) Application number: 97913636.3
(22) Date of filing: 14.11.1997
(51) Int. Cl.: A61K 7/48, A61K 31/20, A61K 31/70, A61K 31/01

(54) **THERAPEUTIC AND COSMETIC COMPOSITIONS, THEIR USE AND METHOD FOR THE PREPARATION THEREOF**
THERAPEUTISCHE UND KOSMETISCHE MITTEL, IHRE VERWENDUNG UND HERSTELLUNGSVERFAHREN
COMPOSITIONS COSMETIQUES ET THERAPEUTIQUES, LEURS UTILISATION ET PROCEDE DE PREPARATION

(30) Priority: 15.11.1996 SE 9604193
(43) Date of publication of application: 05.07.2000
(73) Proprietor: InterHealth AB, 561 51 Huskvarna (SE)
(72) Inventor: KÄRNERUD, Lars, S-560 27 Tenhult (SE); ÖLMESKOG, Stellan, S-578 31 Aneby (SE); NÄSLUND, Ingemar, S-141 39 Huddinge (SE)
(74) Representative: Bergvall-Eftring, Stina Lena
(86) International application number: PCT/SE1997/001921
(87) International publication number: WO 1998/022083

(56) References cited:
- EP-A- 0 366 154
- EP-A- 0 442 420
- WO-A-94/21225
- SE-A- 8 804 241
- US-A- 3 294 639
- US-A- 5 070 081
- US-A- 5 079 003
- PATENT ABSTRACTS OF JAPAN, Vol. 11, No. 69, (C-407); & JP,A,61 227 517 (LION CORP), 9 October 1986.

## Description

The present invention relates to compositions comprising compounds derived from shark liver oil and carbohydrates capable of forming chelate and/or inclusion complexes with the former ones, to the preparation of such compositions, to the use of these compositions for the manufacturing of a pharmaceutical for treating mammals of human or animal origin, as well as to the use of these compositions for the manufacturing of a cosmetic. The invention further relates to a cosmetic method comprising the use of these compositions.

### BACKGROUND OF THE INVENTION

In ancient times shark liver oil, extracted e.g from Greenland shark and from the so called Seamouse, Chimera monstruosa, was used within the traditional medicine for a variety of therapeutic purposes, such as combatting weakness and irritation of the respiratory and digestive organs, gland diseases, bee stings and wound healing etc.

Crude shark liver oil contains squalene, etherlipids (alkylglycerols) and triglycerides. The composition of the oil varies widely from one shark species to another. In some species the oil contains as much as 90% squalene, in others the etherlipids dominate, and in still others the triglycerides.

Due to their varying properties, compounds derivable from the shark liver oil have found applications within various fields of use.

The alkylglycerols have been studied in relation to their beneficial effects on different cancer forms and on the immunosystem. See for example "Alkoxyglycerols and their use in radiation treatment" (Brohult A. Acta Radiol 1963, Suppl 223); "Biochemical effects of alkoxyglycerols and their use in cancer therapy" (Brohult A. et. al. Acta Chem Scand 1970: 24, 730); "Occurrence, synthesis and biological effect of methoxysubstituted glycerol ethers". (Hallgren B. et. al. Progress in chemistry of Fats and other Lipids 1978: 16,45).

In SE 8804241-1 A (Brohult A. and Brohult S.) the peroral use of alkylglycerols for the purpose of treating rheumatic illness is disclosed.

In US 3294639 A (Chalmers W., et. al.) the oral or parenteral use of alkylglycerols, more specifically batyl alcohol, chimyl alcohol and selachyl alcohol, in the treatment of inflammatory diseases in warm-blooded animals is disclosed.

Alkylglycerols also have a disinfectant activity and have been assumed to have a generally beneficial effect on the processes of wound healing.

Squalene, C₃₀H₅₀, is an unsaturated hydrocarbon, constituent of various biological systems and precursor of a number of steroids, e.g. cholesterol. By the hydrogenation of squalene, squalane, C₃₀H₆₂, is obtained. Squalene as well as squalane are natural components of the skin.

Both squalane and squalene have been used within the cosmetics field, as skin lubricants in cosmetic and hygienic preparations and as fixatives in perfumes, in concentrations of up to 50%. However, with squalene there is a stability problem due to its susceptibility to oxidation.

US 5079003 A (Scaffidi A.) discloses creams or lotions comprising emulsions of fish oils, such as shark liver oil, squalane, and squalene that are used as moisturizers, as bases for cosmetics, as hand and body lotions and as sunburn preventives. Compositions are described also comprising numerous other ingredients with therapeutic and synergistic effects on the skin.

Within the therapeutic field, squalene as well as squalane have been used as penetration enhancers of actives, and in adjuvants of vaccines (see e.g. Hoffman SL et. al. Am J Trop Med Hyg 1994 Nov; 51(5): 603-12). As components of oil-emulsion based adjuvants in injection preparations they are considered to have anti-irritating properties (see e.g. Stone HD et. al. Avian Dis 1990 Oct-Dec;34(4): 979-83), and as such they also have shown an immunostimulating effect (Allison AC, Int J Technol Assess Health Care 1994 Winter; 10(1): 107-20). Moreover, they both are deemed to have a proliferation stimulating effect on the dermal as well as the muscular tissues, and squalene protects the dermal lipids from oxidation due to the sun radiation and attack from free radicals of the air (Kohno Y. et. al. Biochim Biophys Acta 1995 Apr 28; 1256(1): 52-6).

The problem inherently associated with the use of slightly water-soluble components in liquid or powdery cosmetic compositions is the quantitative limitation when formulating these components in large amounts and in a uniform state. An inadequate dissolution will give rise to turbidity which will reduce the commercial value of the product, whereas the use of large amounts of solubilizing solvent such as ethanol or polyol will give rise to skin irritation. The slightly water-soluble component is also susceptible to reaction with the other components of the mixture to thereby cause their deterioration and decomposition.

In JP 61-227517 A a solution to this problem is proposed by blending the slightly water-soluble cosmetic component with a cyclodextrin polymer in the form of an inclusion compound thereof with the cyclodextrin polymer. A skin cosmetic having improved feeling in use, free from skin irritation is said to result. A number of examples of slightly water-soluble cosmetic components, among which sqalane, are given. The degree of polymerization of the cyclodextrin polymer is 3-4; the weight ratio of the two components is 0.001-0.5 parts of the slightly water-soluble cosmetic component based on 1 part of the cyclodextrin polymer.

In EP 0366 154 A2 another solution to the same problem is provided in a cosmetic composition comprising an inclusion product having a slightly water-soluble component, e.g. squalane or squalene, with a hydroxyalkylated cyclodextrin formulated therein, the product having improved product characteristics such as useability and stability due to the formulation of an inclusion product with the hydroxyalkylated cyclodextrin. EP 0 366 154 A2 refers to the above mentioned JP 61-227517 A, which proposed to resolve the above outlined problems by the inclusion action of a cyclodextrin polymer, and states that satisfactory results could not be obtained due to the difficulty of obtaining a cyclodextrin polymer per se having a constant quality and also due to the viscosity and quite low water-solubility of the polymer. According to EP 0 366 154 A2 the hydroxyalkylated cyclodextrins, in comparison to the parent cyclodextrins have a significantly enhanced solubility in hydrophilic solvents, such as water, making the use of the solubilizing but skin irritating solvents superfluous, while avoiding the disadvantages of the polycyclodextrins.

Inclusion complexes have been used in the administration of pharmaceutical substances as well as for toxicological studies. Molecules capable of forming inclusion complexes are cyclic carbohydrates such as e.g cyclodextrins and their derivates. In this type of complex, the carbohydrate plays the role of a "host molecule", forming a cavity lodging one or several "guest molecules". Indeed, in a work intitulated "Cyclodextrin Chemistry" (Ed. Springer Verlag) it is stated that the cavity of the cyclodextrin molecule has a calculated diameter of 7 Å and a depth of 7 Å. This may be correlated to the diameter and length of the "guest molecule", which in the case of e.g. the squalane molecule can roughly be estimated to 5 Å and 30-35 Å respectively.

These inclusion complex forming carbohydrate molecules are essentially water soluble. By use thereof, quite hydrophobic molecules such as lipids and paraffins have been encapsulated in the form of water soluble complexes. It has been shown that by use of these molecular complexes, lipophilic substances may be delivered to cells from which, due to their hydrophobic character, they otherwise would be less available or excluded.

Moreover, this type of molecular complex is capable of delivering both hydrophobic and hydrophilic substances having any irriating effects besides their desired therapeutic effect, with considerably less irritation caused than by the direct exposure to the substance.

US 5070081 A (Majid A., et. al.) discloses a method for preparing inclusion complexes of guest molecules and cyclodextrins, in agglomerate form. As examples of guest molecules, there are mentioned flavours, perfumes, agrochemicals and drugs, in liquid as well as solid form, such as citral, citronellal, limonen, peppermint oil, lemon oil, benzaldehyde, allethrins, pyrethroids, salicylic acid, cinnarizine, chloropicrin, phenacetin and tetrahydrocannabinol. The aggregates, which range in size from less than 0.1 mm to more than 1 cm are said to have the advantage of being strong and stable and providing beneficial effects such as controlled release, masking of bad tastes and smells, enhancement of the bioavailability of poorly soluble drugs, etc.

In WO 9421225 A1 disclosure is made of the use of beta-cyclodextrin as a conjugate with a vitamine A derivative in a skin care composition for the therapeutic and prophylactic treatment of ageing symptoms in the skin. The beta-cyclodextrin is present in stoechiometric proportions to the vitamins A derivative, i.e. in a ratio of 1:2 of the vitamins A derivate to the beta-cyclodetrin, and it is said to improve the skin penetration of said derivative.

EP 0 442 420 A1 relates to a cosmetic composition comprising a glutathione/alpha-cyclodextrin and/or beta-cyclodextrin inclusion complex, wherein the complexation has the effect of masking the offensive odor of the glutathione, while the composition retains the beneficial effects of the glutathione, such as a whitening effect and a skin-improving effect.

Also, the gastric tolerability, absorption and pharmacological activity of 4-biphenylacetic acid, a non-steroidal antiinflammatory drug, as an inclusion complex with beta-cyclodextrin or certain chemically modified derivates thereof is reported by Puglisi et. al. (J Pharm Pharmacol 1995 Feb; 47(2):120-3), showing an enhancement of the bioavailability by the use of this sort of complex.

Arima H. et. al. (J Pharm Sci 1992 Nov; 81(11):1119-25) also describe the use of beta-cyclodextrin derivates to enhance the rectal administration and reduce the local irritation of ethyl 4-biphenylylacetate, the prodrug of 4-biphenylacetic acid, in an oily vehicle, and state that the enhancement is due to the fact that beta-cyclodextrin increases the release rate of the prodrug from the vehicle and stabilizes the same in the rectal lumen, and that the prodrug was partly absorbed in the form of a complex with the beta-cyclodextrin.

Trucco M. et. al. (Headache 1992 Jan;32(1):39-40) describe a case of medical use of a complex of the drug piroxicam and beta-cyclodextrin by oral administration wherein a good gastric tolerability is obtained. This finding is later confirmed by Cadel S. et. al. (Acta Physiol Hung 1990; 75 Suppl:45-6).

The protective effect of beta-cyclodextrin against the local tissue toxicity of a neuroleptic, chlorpromazine, by intramuscular injection to mice, was investigated by Irie T. et. al., (J Pharmacobiodyn 1983 Oct;6(10):790-2). This effect was ascribed to the inclusion complex formed between the two compounds, and the authors propose beta-cyclodextrin complexation to be particularly useful for reducing the local toxicity of phenotiazine neuroleptics without altering the pharmacological efficacy.

Szejtli J. et. al. (Pharmazie 1981 Apr;36(4):283-6) studied the influence of complexation of salicylic acid with beta-cyclodextrin on resorption and side-effects of salicylic acid in rats, and concluded that the complex formation decreases the stomach irritating effect of salicylic acid.

US patent 5,321,014 discloses a method of preparing a complex of cyclodextrin and an alkane, alkene, alkyne, aromatic compound, etc. Delivery of these complexes to prokaryotic and eukaryotic cells, tissues and organs, in vitro and in vivo, provides a method of determining the toxic, genotoxic and mitogenic effects of these compounds.

The other type of complex usable according to the invention, i.e. the chelate complex, may be obtained by the use of other carbohydrates, such as saccharides and oligosaccharides. In these types of complexes, a "guest molecule" may be surrounded by one or several "host molecules", the so called sequestrants, which due to their spatial configuration in combination with their functional groups retain the former one.

We herein thus refer to two types of complexes, i.e. the chelate complex versus the inclusion complex, however, it should be realized that there could also exist intermediary species between these two extremes.

The present inventors now have found, that by combining a component comprising compounds extractable from shark liver oil with an inclusion or chelate complex forming component, a pharmaceutical or cosmetic having a surprising enhanced effect, compared to the use of either of the components alone, can be obtained. Such is the case for a preparation to protect the skin from harmful radiations, either from the sun or from artificial radiation sources such as by radiation therapy of a cancer, i.e. radiotherapy. A therapeutic composition actually applied for this latter purpose is disclosed in EP 0640 346 A1, an ointment based on sulphated sugars, under the tradename of BM ointment. It has been shown that the sulphated sugars of the above mentioned application besides an effect on e.g. peptic ulcers by oral administration, have a beneficial prophylactic and curing effect on different states of inflammation of e.g. the skin, by topical administration. This beneficial effect has been used in radiotherapy, where the electrons and photons in passing through the skin of the patient cause burning symptoms, such as redness and itching of the skin, in a dose dependent way, which radiation side effects may last for several weeks, and in some cases may be even worse than a burn. Although the linear accelerators used nowadays have the advantage of giving lower doses to the skin, the problem still remained unsolved until the commercial BM ointment based on the above mentioned patent became available. Said commercial ointment is very efficient, giving a substantial reduction of reddening and itchiness.

### GENERAL DESCRIPTION OF THE INVENTION

The invention is based on the combination of a component, as defined in the appended claims, derived from an extract of crude shark liver oil, or a synthetic equivalent of such a component, and a carbohydrate, also as defined in the claims, capable of forming an inclusion and/or chelate complex with the former one.

The compositions of the invention accordingly are characterized in that they comprise as active ingredients
(i) one or several components derived from an extract of crude shark liver oil, selected from the group comprising refined shark liver oil or a fraction thereof, squalane, squalene and alkylglycerols, and/or the synthetic equivalents of these;
(ii) one or several carbohydrates forming inclusion and/or chelate complex with said component (i), selected from the group comprising beta-cyclodextrin, alpha-cyclodextrin, gamma cyclodextrin, and delta-cyclodextrin; and optionally
(iii) one or several non-shark liver oil derived therapeutically or cosmetically active agents.

A method of preparing the compositions of the invention also is provided, characterized by admixing (ii) contained in a hydrophilic medium with (i) contained in a hydrophobic medium, agitating and optionally heating until a homogeneous emulsion is formed, and optionally admixing (iii) into said homogeneous emulsion.

The new compositions of the invention have a prophylactic and curing effect on different states of inflammation and disorder of the skin, as well as a generally beneficial effect on the state of the skin. The compositions also may be formulated for the treatment of wounds of different origins.

By their beneficial effect on the skin in association with the penetration enhancing effect, the compositions of the present invention when incorporating additionally other, non-shark liver oil derived actives, i.e. active agents not being derived from shark liver oil, will allow the topical administration of these other actives while preventing local irritation from the same on the skin.

Moreover the use of the compositions of the invention comprising such non-shark liver oil derived actives in some cases will provide an alternative to the systemic administration, by replacing e.g. oral administration by topical administration.

The use of a composition according to the invention for the preparation of a pharmaceutical for topical administration to a mammal of human or animal origin also is provided.

The compositions of the invention furthermore may be of utility within the cosmetic field i.a. to prevent the ageing of the skin by combatting the formation of wrinkles.

Finally, the invention provides the use of a cosmetic composition according to the invention in a cosmetic method.

### DETAILED DESCRIPTION OF THE INVENTION

The first active component of the invention is derived from an extract of crude shark liver oil, selected from the group comprising refined shark liver oil or a fraction thereof, squalane, squalene and alkylglycerols, and/or the synthetic equivalents of these.

Suitable examples of alkylglycerols are chimyl alcohol, batyl alcohol and selachyl alcohol as well as their fatty acid esters.

The selection of the compound or compounds to be included as the first component depends on the intended purpose of the composition. For example, in a preferred embodiment of the invention, a composition to be applied in radiotherapy to protect the skin from the harmful effects from the radiation will comprise squalane as the active first component.

In another embodiment, a composition to be applied preventively to the skin to protect it from the harmful effects of the sun radiation will also comprise squalene.

In still another embodiment alkylglycerols may be included as disinfectants having a generally beneficial effect on a wound healing process.

The second component of the composition of the invention is comprised of one or several carbohydrates forming an inclusion and/or chelate complex with the first component.

The composition of the invention contains the two essential components, i.e. the component derived from crude shark liver oil, or its synthetic equivalent, and the inclusion complex forming carbohydrate, in a total amount ranging anywhere between a very low value, such as 1%, up to a very high value, such as 100%.

Ideally, the relative amounts of the two components are such as to give a composition wherein essentially all of the first component is complexed with essentially all of the second component. However, the forming of the inclusion and/or chelate complex most probably being an equilibrium reaction, it may be beneficial to add the first component, i.e. the shark liver oil derived component, in a certain excess to the second component, i.e. the complex forming component, or, on the contrary, the second component in excess to the first one.

In general, a molar ratio ranging from 15:1 to 1:15, preferably 15:1 to 3:5, also preferably 5:3 to 3:5, and most preferably 3:1, of the first component to the second one would be suitable.

In a preferred embodiment of the invention, a composition such as for application on skin to be irradiated in a cancer radiotherapy may comprise 6% squalane and 6% beta-cyclodextrin by weight of the total composition, i.e. in a weight ratio of 1:1 to each other. Taking account of the water content of the beta-cyclodextrin, amounting to eight moles of water in each mole of beta-cyclodextrin, the mole weight of this component will be 1279g, whereas that of squalane is 422g. The molar ratio of the two components in the above ointment thus is 3:1 of squalane to beta-cyclodextrin.

The compositions of the invention optionally may contain combinations of the herein defined active components derived from shark liver oil as well as other, non-shark liver oil derived therapeutically or cosmetically active agents, to be selected by the man skilled in the art in view of the aimed purpose of the formulation. As an example, an analgesic may be incorporated into a therapeutic compositon of the present invention, since often skin disorders as well as skin wounds of different origins are accompanied by a sensation of pain.

Alcohol soluble as well as water soluble analgesics are suitable according to the invention. In the case of alcohol soluble analgesics, a small amount of an alcohol such as ethanol may be used to solubilize the analgesic before incorporating it into the composition of the invention.

Examples of suitable analgesics are derivates of salicylic acid, such as methyl salicylic acid, acetyl salicylic acid, diethylamino salicylic acid, as well as the pharmaceutically acceptable salts thereof, paracetamol, indomethacin, sulindac, diclofenac, ketorolac, piroxicam, tenoxicam, ibuprofen, naxoprofen, ketoprofen, nabumeton, azapropazone, auranofin, lidocaine, prilocaine, codeine and other opiates, etc.

Other examples of suitable non-shark liver oil derived active agents which may be included in the inventive compositions are antifungals, antibiotics and antivirals. In this case, the compostion of the invention will provide an enhanced penetration of the actives not only into the attacked cells of the treated subject, but also an enhanced penetration of the antimicrobial into the infectious agent.

Examples of suitable antifungals are nystatin, clotrimazole, miconazole, econazole, ketoconazole, bifonazole.

Examples of suitable antibiotics are terbinafine, amorolfine, fusidic acid, neomycin, gentamicin, mupirocine.

Examples of suitable antivirals are podophyllotoxin and metronidazole.

As already mentioned herein above, the beneficial effect on the skin in association with the penetration enhancing effect may also be used in compositions furthermore comprising another active agent to be delivered into the body of a person suffering from a state susceptible of being alleviated by this latter active. The well-known problem of formulating compositions comprising slightly water soluble components which additionally in themselves may be irritant to the skin here finds it solution.

As an example, by a composition comprising, in addition to the active agent to be delivered, squalane and a cyclodextrin topical administration will be possible in cases where formerly systemic administration was used or where the active had an irritating effect on the skin. The beneficial effect on the skin of the squalane here acts to reduce irritation while the cyclodextrin molecule enhances penetration of the actives.

This topical administration will be of great value e.g. in the treatment of states responsive to treatment with analgesics, since it is well-known that orally administered analgesics may give rise to a multitude of negative side effects, such as gastrointestinal disorders, abdominal pain and cramps, dyspepsia, nausea, vomiting, diarrhoea, etc.

By topical administration of the analgesic as a local treatment, the passage of the analgesic through the gastrointestinal tract will be avoided and consequently the above mentioned specific side effects in relation to this passage will not be encountered. Moreover, the necessary dosage of the analgesic to obtain the desired therapeutic effect will be lower, which further reduces the risk of negative side effects.

Examples of states of pain that may be treated in this way are: muscular damage and pain, tennis elbow, migraine and tension ache.

Some cases of severe migraine may be accompanied by a dysfunction of the gastrointestinal absorption, making the oral use of conventional analgesics impracticable. In such cases of dysfunction an additional benefice will then be provided by the topical administration without necessity of gastrointestinal absorption.

The concentration of the analgesic in the composition of the invention, e.g. in the form of an ointment, preferably is such that 15 g of the ointment correspond to the recommended daily dosage of the analgesic. However, in cases where a reinforcment of the analgesic effect is required, as little as 5 g of the ointment may correspond to the recommended daily dosage of the analgesic. On the other hand, when a lower analgesic effect is required, up to 50 g of the ointment may correspond to the recommended daily dosage of the analgesic.

An example of an analgesic which may be incorporated into a composition of the invention is ketoprofen. The recommended maximum daily dosage of this analgesic is 0.375 g. This would correspond to a concentration of 2.5% by weight in 15 g of ointment, whereas an ointment having a reinforced analgesic effect as exemplified above would correspond to a concentration of 7.5% by weight, the lower effect corresponding to a concentration of 0.75% by weight.

A lotion, such as an after sun or sunburn lotion, also may comprise a low level of an analgesic.

The compositions of the invention also may contain other optional components, conventional to the field of the art, such as formulation aids, e.g. preservatives.

The composition of the invention will be in a form suitable for topical application, such as i.a. an ointment, a creme, a gel, a milk, a lotion, a powder and a liquid emulsion.

The pharmaceutical preparation may e.g. be an ointment to be applied on the skin as well as in the oral cavity, vaginally, rectally, or nasally.

The compositions of the invention may be used for a variety of conditions, prophylactically or curatively. Examples of the fields of use of the compositions of the invention include the preventive application on skin to be irradiated in a radiation therapy of a cancer, the curative application on skin burnt by excessive exposure to sun radiation, the preventive application on skin in view of exposure to the sun radiation, the application in wound healing, healing of burns, of abrasions. Other examples of use of the compositions of the invention are for the treatment of skin disorders such as different forms of allergy, psoriasis, eczema and atopic dermatitis.

Within the cosmetic field the compositions of the invention may be used to reduce or prevent the formation of wrinkles, in combatting the ageing of the skin. They may be formulated as an ointment, a creme, a gel, a milk, a lotion, a powder and a milk.

The invention also provides a method of preparing the compositions of the invention. This method comprises admixing a hydrophilic medium solution or dispersion of the carbohydrate component with a lipophilic medium solution or dispersion of the shark liver oil component, agitating and, if necessary, heating until a homogeneous emulsion of the two components is formed, and optionally adding a non-shark liver oil derived active to the homogeneous emulsion.

The heating temperature will be the one giving, in combination with the agitation, a homogeneous emulsion of the components of the composition. This can range e.g. from just above room temperature, such as 30°C, to an upper limit, of e.g 90°C. Obviously, the upper limit of the heating temperature range is set by the boiling point of the mixture, but care also should be taken not to decompose unduly the components of the mixture by excessive heating.

The hydrophilic medium used to dissolve the carbohydrate may comprise water or a pharmaceutically and/or cosmetically acceptable alcohol such as ethanol, propylene glycol, and various suitable mono-, di- and trihydric alcohols, conventional to the art, as well as a mixture of any of these.

The lipophilic medium used to dissolve the lipophilic, i.e. hydrophobic component, may comprise a pharmaceutically and/or cosmetically acceptable vegetable oil, or any lipidic creme basis such as conventionally used within the field of the art.

Depending on the relative amounts of the aqueous and the oily phases, an ointment, a creme, a gel, a milk or a lotion will be obtained, as will be known to the man skilled in the art.

The invention will now be further illustrated by way of the non-limiting examples herein below.

### EXAMPLE 1

An ointment is prepared from the following ingredients:

| Ingredients | % by weight |
|---|---|
| Squalane | 6.0 |
| Cyclodextrin | 6.0 |

| Creme base (lipophilic medium): | |
|---|---|
| Cremeol™ FR 36 | 5.2 |
| Cremeol™ FR 57 | 5.2 |
| Cremeol™ SH | 3.7 |
| Glucamate™ SSE-20 | 1.8 |
| Glucate™ SS | 1.2 |

| Aqueous phase (hydrophilic medium): | |
|---|---|
| Water | 65.75 |
| Sorbic acid | 0.15 |
| Corn PO4 PH "B"™, | 5.0 |

Squalane is mixed with the lipophilic medium, i.e. the creme base, and, separately, cyclodextrin is mixed with the hydrophilic medium, i.e. the aqueous phase. Parts or all of the squalane may be added to the aqueous phase together with the cyclodextrin to develop the inclusion complex. Both phases are separately heated to 75°C under agitation until homogeneous. The aqueous phase is slowly poured into the oily phase under agitation. The mixture having cooled to about 40°C, Corn PO4 PH "B"™ is added. The finished mixture then is cooled to 20°C and packaged.

This ointment may be used e.g. as a protection of the skin in radiation therapy as well as in the treatment of some autoimmune diseases. The ointment also has a generally beneficial effect on skin which makes it useful e.g. as a cosmetic to prevent ageing of the skin.

### EXAMPLE 2

A lotion is prepared from the following ingredients:

| Ingredients | % by weight |
|---|---|
| Squalane | 6.0 |
| Cyclodextrin | 6.0 |

| Oily phase (Lipophilic medium): | |
|---|---|
| Tefose™ 2561 | 10.0 |
| Cremeol™ HF52 | 2.5 |
| Cremeol™ HF62 | 2.5 |
| Ascorbyl palmitate | 0.1 |

| Aqueous phase (Hydrophilic medium): | |
|---|---|
| Water | 67.64 |
| Sorbic acid | 0.15 |
| | |
| Corn PO4 PH "B"™ | 5.0 |

Squalane is mixed with the lipophilic medium while agitating and heating to 75°C. Separately, sorbic acid is mixed with the water, while agitating and heating to 75°C until complete dissolution thereof. When a homogeneous aqueous solution of the sorbic acid is obtained, the cyclodextrin is admixed. The aqueous phase is slowly poured into the oily phase under agitation. The agitation is maintained while the mixure is allowed to cool to 50°C, at which point the Corn PO4 PH "B"™ is added.

This lotion may be used on skin burnt by excessive exposure to the sun.

### EXAMPLE 3

An ointment is prepared from the following ingredients:

| Ingredients | % by weight |
|---|---|
| Squalane | 3.0 |
| Cyclodextrin | 6.0 |

| Oily phase (lipophilic medium): | |
|---|---|
| Cremeol™ FR 36 | 5.6 |
| Cremeol™ SH | 2.0 |
| Ascorbyl palmitate | 0.01 |
| Glucamate™ SSE-20 | 1.8 |
| Glucate™ SS | 1.2 |

| Aqueous phase (hydrophilic medium): | |
|---|---|
| Water | 70.74 |
| Sorbic acid | 0.15 |
| | |
| Ethanol 96% | 2.0 |
| Ketoprofen | 2.5 |
| | |
| Corn PO4 PH "B"™ | 5.0 |

Squalane is mixed with the lipophilic medium, i.e. the oily phase which is then heated to 75°C while agitating. Separately, the sorbic acid is added to the water and the aqueous phase is heated to 75°C while agitating until all of the sorbic acid is dissolved. Then the cyclodextrin is admixed into the aqueous phase. The aqueous phase is slowly poured into the oily phase under agitation. The ketoprofen dissolved in ethanol is added. The ointment is allowed to cool to about 50°C while maintaining the agitation, and at this point Corn PO4 PH "B"™ is added. The finished mixture finally is cooled to 20°C and packaged.

This ointment may be used in the treatment of different states of pain, such as tennis elbow.

### EXAMPLE 4

An ointment is prepared from the following ingredients:

| Ingredients | % by weight |
|---|---|
| Squalane | 3.0 |
| Cyclodextrin | 6.0 |

| Creme base (lipophilic medium): | |
|---|---|
| Cremeol™ FR 36 | 5.6 |
| Cremeol™ SH | 2.0 |
| Ascorbyl palmitate | 0.01 |
| Glucamate™ SSE-20 | 1.8 |
| Glucate™ SS | 1.2 |

| Aqueous phase (hydrophilic medium): | |
|---|---|
| Water | 65.24 |
| Sorbic acid | 0.15 |
| Ethanol 96% | 5.0 |
| Paracetamol | 5.0 |
| Corn PO4 PH "B"™, | 5.0 |

The ointment of this example is prepared following the procedure of example 3, but replacing the ketoprofen with paracetamol.

This ointment may be used to alleviate different states of pain.

### EXAMPLE 5

A sunburn lotion is prepared from the following ingredients:

| Ingredients. | % by weight |
|---|---|
| Squalane | 6.0 |
| Cyclodextrin | 6.0 |

| Oily phase (Lipophilic medium): | |
|---|---|
| Tefose™ 2561 | 10.0 |
| Cremeol™ FR36 | 5.0 |
| Ascorbyl palmitate | 0.1 |

| Aqueous phase (Hydrophilic medium): | |
|---|---|
| Water | 65.29 |
| Sorbic acid | 0.15 |
| | |
| Paracetamol | 0.5 |
| Ethanol 96% | 2.5 |
| | |
| Corn PO4 PH "B"™ | 5.0 |

Squalane is mixed with the lipophilic medium while agitating and heating to 75°C. Separately, sorbic acid is mixed with the water, while agitating and heating to 75°C until complete dissolution thereof. When a homogeneous aqueous solution of the sorbic acid is obtained, the cyclodextrin is admixed. The aqueous phase is slowly poured into the oily phase under agitation. The paracetamol dissolved in the ethanol is added. The agitation is maintained while the mixure is allowed to cool to 50°C, at which point the Corn PO4 PH "8"™ is added.

This lotion may be used on skin burnt by excessive exposure to the sun.

### EXAMPLE 6

An ointment is prepared from the following ingredients:

| Ingredients | % by weight |
|---|---|
| Refined shark liver oil | 9.0 |
| Cyclodextrin | 6.0 |

| Creme base (lipophilic medium): | |
|---|---|
| Tefose™ 2561 | 12.0 |
| Ascorbyl palmitate | 0.05 |

| Aqueous phase (hydrophilic medium): | |
|---|---|
| Water | 67.80 |
| Glycerol | 5.0 |
| Sorbic acid | 0.15 |

The refined shark liver oil is mixed with the lipophilic medium, i.e. the oily phase which is then heated to 75°C while agitating. Separately, the sorbic acid is added to the water mixed with the glycerol, and the aqueous phase is heated to 75°C while agitating until all of the sorbic acid is dissolved. Then cyclodextrin is admixed into the aqueous phase. The aqueous phase is slowly poured into the oily phase under agitation. The ointment is allowed to cool to about 20°C while maintaining the agitation.

This ointment may be used on different forms of wounds.

### EXAMPLE 7

| Ingredients | % by weight |
|---|---|
| Squalane | 3.0 |
| Cyclodextrin | 6.0 |

| Creme base (lipophilic medium): | |
|---|---|
| Cremeol™ FR 36 | 5.6 |
| Cremeol™ SH | 2.0 |
| Ascorbyl palmitate | 0.01 |
| Glucamate™ SSE-20 | 1.8 |
| Glucate™ SS | 1.2 |

| Aqueous phase (hydrophilic medium): | |
|---|---|
| Water | 68.24 |
| Sorbic acid | 0.15 |
| | |
| Ethanol 96% | 5.00 |
| Miconazole | 2.0 |
| | |
| Corn PO4 PH "B"™, | 5.0 |

The ointment of this example is prepared following the procedure of example 3, but replacing the ketoprofen with miconazole.

This ointment may be used in the treatment of different fungal infections.

The chemical definitions of the substances referred to by their trademarks are as follows:
Cremeol™ FR 36: C16-C18 mono-, di- and triglycerides;
Cremeol™ FR 57: C16-C18 mono-, di- and triglycerides;
Cremeol™ SH: Shorea Butter CAS no. 91770-65-9 (Botanical classification Shorea stenoptera);
Cremeol™ HF52: Triglycerides;
Cremeol™ HF62: Triglycerides;
Glucamate™ SSE-20: PEG-20 methyl glucose sesquistearate;
Glucate™ SS: methyl glucose sesquistearate;
Tefose™ 2561: PEG-6 stearate, 50-70.2%; Ceteth-20 10-24.9%;
glyceryl stearate 5-9.9%; Steareth-20 5-9.9%;
CORN PO4 PH "B"™: Cross-linked di-starchphosphate based on corn starch.

The Cremeol™ series is commercially available from Aarhus Oljefabrik A/S, of Aarhus, Denmark.

The Glucate™ SS and Glucamate™ SSE-20 are commercially available from Amerchol Corporation, of Edison, USA.

Tefose™ 2561 is commercially available from Gattefosse, of Saint-Priest, France.

CORN PO4 PH "B"™ is commercially available from Dr. HAUSER GMBH, of Garmisch-Partenkirchen, Germany.

### CLINICAL TESTS

### Ointment

The ointment of the example 1 has been tested clinically on the skin of human subjects exposed to electron beam radiation during a radiotherapy of a cancer, performed at Radiumhemmet, in Stockholm, Sweden. In the tests, the subjects received the composition of the invention on half of the irradiated area and a commercially available ointment, the BM ointment, on the other half. The ointment of the invention showed comparable and in two cases even better results compared to the commercial ointment.

In a previous similar experiment, compositions comprising squalane but no cyclodextrin were used in comparison to the BM ointment. This squalane ointment is commersially accessible as Jurosept™, from Pronova Biocare a.s. of Norway.

The results obtained in this comparative experiment clearly showed that the squalane ointment had some however not sufficient inhibiting effect on injuries caused by the radiation.

The ointment according to example 1 moreover has been used in other therapeutic treatments, with good results:

In a pilot study on the therapeutic effect of the ointment on autoimmune diseases, a girl suffering from lichen sclerosus has been treated by application of the ointment twice a day for a period of 6 weeks. A clear improvement of the state has been noted. Treatment is still going on.

In another case, a girl aged 11 years suffered from an eczema resisting therapy with conventional methods, i.e. cortisone or urea based ointments. The eczema was exacerbated when exposed to sun radiation. Treatment with the above ointment was initiated on a restricted area of the skin, on one heel. When applying the ointment twice a day, the eczema completely disappeared in the treated area within one month. Treatment was then pursued on other parts of the body, and is still going on.

### Lotion

The lotion of example 2 was applied on skin burnt by excessive exposure to the sun. The lotion was applied in the evening of the day of excessive exposure to the sun. An essential alleviation of symptoms such as redness and itching and burning sensations was obtained.

## Claims

1. A composition **characterized in that** it comprises as active ingredients
(i) one or several components derived from an extract of crude shark liver oil, selected from the group comprising refined shark liver oil or a fraction thereof, squalane, squalene and alkylglycerols, and/or the synthetic equivalents of these;
(ii) one or several carbohydrates forming inclusion and/or chelate complex with (i), selected from the group comprising beta-cyclodextrin, alpha cyclodextrin, gamma-cyclodextrin, delta-cyclodextrin; and optionally
(iii) one or several non-shark liver oil derived therapeutically or cosmetically active agents.

2. The composition according to claim 1 **characterized in that** (i) and (ii) are present in a molar ratio of 15:1 to 1:15 of (i) to (ii).

3. The composition according to claim 1 or 2 **characterized in that** (i) and (ii) are present in a molar ratio of 5:3 to 3:5 of (i) to (ii).

4. The composition according to any of the previous claims, **characterized in that** (i) and (ii) are present in a molar ratio of 3:1 of (i) to (ii).

5. The composition according to any of the previous claims **characterized in that** (i) is squalane.

6. The composition according to any of the previous claims **characterized in that** (ii) is beta-cyclodextrin.

7. The composition according to any of the previous claims in the form of an ointment, a creme, a gel, a lotion, a powder or a milk.

8. The composition according to any of the previous claims for the use as a pharmaceutical for treating mammals of human or animal origin.

9. The composition according to the claim 8 comprising a therapeutically active agent selected from analgesics, antifungals, antibiotics and antivirals.

10. The composition according to any of the claims 1-7 for the use as a cosmetic.

11. A method of preparing the composition according to any of the previous claims **characterized by** admixing (ii) contained in a hydrophilic medium with (i) contained in a hydrophobic medium, agitating and optionally heating until a homogeneous emulsion of (i) and (ii) is formed, and optionally admixing (iii) into said homogeneous emulsion.

12. A method of combatting the ageing of the skin by the application thereto of a composition according to claim 10 to prevent or reduce the formation of wrinkles.

13. Use of a composition according to any of the claims 1-7 for the preparation of a pharmaceutical for topical administration to a mammal of human or animal origin.

14. Use of a composition according to any of the claims 1-7 for the preparation of a cosmetic to be applied to the skin to combat the ageing thereof by preventing or reducing the formation of wrinkles.

## Patentansprüche

1. Zusammensetzung, **dadurch gekennzeichnet, dass** sie als Wirkstoffe umfasst:
(i) eine oder mehrere Komponenten, die von einem Extrakt aus rohem Haileberöl herrühren, ausgewählt aus der Gruppe umfassend raffiniertes Haileberöl oder eine Fraktion davon, Squalan, Squalen und Alkylglycerine, und/oder die synthetischen Äquivalente von diesen;
(ii) ein oder mehrere Kohlenhydrate, die einen Einschluss- und/oder Chelatkomplex mit (i) bilden, ausgewählt aus der Gruppe umfassend beta-Cyclodextrin, alpha-Cyclodextrin, gamma-Cyclodextrin, delta-Cyclodextrin; und gegebenenfalls
(iii) ein oder mehrere nicht von Haileberöl herrührende therapeutisch oder kosmetisch wirksame Mittel.

2. Zusammensetzung nach Anspruch 1, **dadurch gekennzeichnet, dass** (i) und (ii) in einem Molverhältnis von 15 : 1 bis 1 : 15 von (i) zu (ii) vorhanden sind.

3. Zusammensetzung nach Anspruch 1 oder 2, **dadurch gekennzeichnet, dass** (i) und (ii) in einem Molverhältnis von 5 : 3 bis 3 : 5 von (i) zu (ii) vorhanden sind.

4. Zusammensetzung nach einem der vorangehenden Ansprüche, **dadurch gekennzeichnet, dass** (i) und (ii) in einem Molverhältnis von 3 : 1 von (i) zu (ii) vorhanden sind.

5. Zusammensetzung nach einem der vorangehenden Ansprüche, **dadurch gekennzeichnet, dass** (i) Squalan ist.

6. Zusammensetzung nach einem der vorangehenden Ansprüche, **dadurch gekennzeichnet, dass** (ii) beta-Cyclodextrin ist.

7. Zusammensetzung nach einem der vorangehenden Ansprüche in Form einer Salbe, einer Creme, eines Gels, einer Lotion, eines Pulvers bzw. Puders oder einer Milch.

8. Zusammensetzung nach einem der vorangehenden Ansprüche zur Verwendung als Pharmazeutikum zum Behandeln von Säugem menschlicher oder tierischer Herkunft.

9. Zusammensetzung nach Anspruch 8, umfassend ein therapeutisch wirksames Mittel, ausgewählt aus Analgetika, Antimykotika, Antibiotika und antiviralen Substanzen.

10. Zusammensetzung nach einem der Ansprüche 1 - 7 zur Verwendung als Kosmetikum.

11. Verfahren zum Herstellen der Zusammensetzung nach einem der vorangehenden Ansprüche, **gekennzeichnet durch** Vermischen von (ii), das in einem hydrophilen Medium enthalten ist, mit (i), das in einem hydrophoben Medium enthalten ist, Rühren und gegebenenfalls Erwärmen, bis eine homogene Emulsion von (i) und (ii) gebildet ist, und gegebenenfalls Zumischen von (iii) in diese homogene Emulsion.

12. Verfahren zum Bekämpfen der Alterung der Haut durch die Anwendung einer Zusammensetzung nach Anspruch 10 darauf, um die Bildung von Falten zu verhüten oder zu verringern.

13. Verwendung einer Zusammensetzung nach einem der Ansprüche 1 - 7 zum Herstellen eines Pharmazeutikums für eine topische Verabreichung an einen Säuger menschlicher oder tierischer Herkunft.

14. Verwendung einer Zusammensetzung nach einem der Ansprüche 1 - 7 zum Herstellen eines Kosmetikums, das auf die Haut aufgetragen werden soll, um deren Alterung durch Verhütung oder Verringerung der Bildung von Falten zu bekämpfen.

## Revendications

1. Une composition **caractérisée en ce qu'**elle comprend comme ingrédients actifs
(i) un ou plusieurs composés dérivés d'un extrait d'huile de foie de requin brute choisis parmi le groupe comprenant l'huile de foie de requin raffinée ou une fraction de celle-ci, le squalane, le squalène et les alkylglycérols et/ou les équivalents synthétiques de ceux-ci ;
(ii) un ou plusieurs glucides formant des complexes d'inclusion et/ou des complexes de chélation avec (i), choisi parmi le groupe comprenant les beta-cyclodextrine, alpha-cyclodextrine, gamma-cyclodextrine, delta-cyclodextrine, et optionnellement ;
(iii) un ou plusieurs agents actifs sur le plan thérapeutique ou cosmétique qui ne soient pas dérivés d'huile de foie de requin.

2. La composition selon la revendication 1 **caractérisée en ce que** (i) et (ii) sont présents dans un rapport molaire de 15:1 à 1:15 de (i) à (ii).

3. La composition selon la revendication 1 ou 2 **caractérisée en ce que** (i) et (ii) sont présents dans un rapport molaire de 5:3 à 3:5 de (i) à (ii).

4. La composition selon l'une quelconque des revendications précédentes **caractérisée en ce que** (i) et (ii) sont présents dans un rapport molaire de 3:1 de (i) à (ii).

5. La composition selon l'une quelconque des revendications précédentes **caractérisée en ce que** (i) est le squalane.

6. La composition selon l'une quelconque des revendications précédentes **caractérisée en ce que** (ii) est la beta-cyclodextrine.

7. La composition selon l'une quelconque des revendications précédentes sous la forme d'un onguent, d'une crème, d'un gel, d'une lotion, d'une poudre ou d'un lait.

8. La composition selon l'une quelconque des revendications précédentes pour utilisation comme produit pharmaceutique pour traiter les mammifères d'origine humaine ou animale.

9. La composition selon la revendication 8 comprenant un agent actif sur le plan thérapeutique choisi parmi les analgésiques, les antifungiques, les antibiotiques et les antiviraux.

10. La composition selon l'une quelconque des revendications 1 - 7 pour être utilisée comme un produit cosmétique.

11. Un procédé de préparation de la composition selon l'une quelconque des revendications précédentes **caractérisé par** les étapes de mélange de (ii) contenu dans un milieu hydrophile avec (i) contenu dans un milieu hydrophobe, l'agitation et optionnellement le chauffage jusqu'à l'obtention d'une émulsion homogène de (i) et (ii) et optionnellement l'incorporation de (iii) dans ladite émulsion homogène.

12. Un procédé pour lutter contre le vieillissement de la peau par l'application sur celle-ci d'une composition selon la revendication 10 pour prévenir ou réduire la formation des rides.

13. L'utilisation d'une composition selon l'une quelconque des revendications 1 - 7 pour la préparation d'un produit pharmaceutique pour l'administration topique à un mammifère d'origine animale ou humaine.

14. L'utilisation d'une composition selon l'une quelconque des revendications 1 - 7 pour la préparation d'un produit cosmétique destiné à être appliqué sur la peau pour lutter contre le vieillissement de celle-ci par la prévention ou la réduction de la formation de rides.
